(19) **Europäisches Patentamt European Patent Office Office européen des brevets**

(11) **EP 4 512 311 A1**

(12) **EUROPEAN PATENT APPLICATION**

(43) Date of publication:
26.02.2025 Bulletin 2025/09

(51) International Patent Classification (IPC):
*A61B 5/00* (2006.01)   *A61B 5/318* (2021.01)

(21) Application number: 24195176.3

(22) Date of filing: 19.08.2024

(52) Cooperative Patent Classification (CPC):
**A61B 5/7203; A61B 5/318; A61B 5/7225;**
**A61B 5/7239; A61B 5/725;** A61B 5/7207

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 22.08.2023 US 202363533978 P

(71) Applicant: **Drägerwerk AG & Co. KGaA**
**23558 Lübeck (DE)**

(72) Inventor: **ZHANG, Ruwen**
**Andover, 01810 (US)**

(74) Representative: **Haseltine Lake Kempner LLP**
**One Portwall Square**
**Portwall Lane**
**Bristol BS1 6BH (GB)**

(54) **FAST AUTOBLOCKING FOR PHYSIOLOGICAL WAVEFORM DISPLAY**

(57)     A method for autoblocking a physiological waveform display to mitigate baseline wandering, includes: extracting a low resolution preliminary baseline estimate from an input physiological waveform; extracting a high resolution deviation mean from the input physiological waveform; subtracting the low resolution preliminary baseline estimate from the high resolution deviation mean to extract a mean deviation of the input physiological waveform; determining a weight factor positively correlated to the mean deviation; applying the weight factor to the mean deviation of the input physiological waveform; applying the difference between 1 and the weight factor to the preliminary baseline estimate; combining the weighted mean deviation of the input physiological waveform with the weighted preliminary baseline estimate to extract an adjusted baseline estimate; and subtracting the adjusted baseline estimate from the input physiological waveform to obtain an autoblocked physiological waveform for display.

FIG. 1

**Description**

TECHNICAL FIELD

The present disclosure relates generally to the field of medical monitoring of physiological parameters in a patient and, more particularly, to monitoring and displaying physiological parameters representing a patient's condition.

BACKGROUND

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Conventional, modern medicine frequently includes measurement and/or monitoring of a patient's physiological condition. Commonly known physiological parameters that are measured and/or monitored might include, for example and without limitation, blood pressure, body temperature, vision acuity, quantities of various substances in the blood, etc. Because of the value imparted by this information, the art of medical monitoring has developed an array of instruments and procedures for acquiring this kind of information.

For example, one well-known measurement/-monitoring technique is an "electrocardiogram" ("ECG"). Electrocardiograms are commonly used to monitor patients' heart conditions as well as to detect or predict cardiac events and conditions. In clinical settings, ECG signals representative of a patient's condition are captured in waveforms and analyzed by physiological monitoring devices. However, ECGs are also useful for acquiring other kinds of medical information besides cardiac events and conditions.

An ECG graphs voltage acquired from a person's body over time. The voltages represent electrical activity of the heart that cause the heart to beat. To acquire the voltages, sensors, or "electrodes", are placed at selected points on the person's body. The number and location of the electrodes are fairly standardized but may vary depending on the type of medical information that is of interest. For example, in one common nomenclature, the sensors are electrically connected to the physiological monitoring device by cables. The sensors and cables together are then referred to as "leads".

Physiological parameter data, such as ECG data, is frequently displayed on a patient monitor as waveforms. In the present context, the patient monitor may also be referred to as a "physiological waveform monitor" and the physiological data may also be referred to as a "physiological parameter waveform". The ECG waveforms are but one example of a "physiological parameter waveform". Physiological parameter waveforms can also represent other physiological parameters such as those set forth above-e.g., for example and without limitation, blood pressure, body temperature, vision acuity, quantities of various substances in the blood, etc.

Physiological parameter waveform display can be subject to problems associated with data collection and/or processing. One issue is known as "baseline wander", which can be a low frequency artifact in the data that is not associated with the patient's condition. For example, in an ECG display, baseline wander may be attributable to factors such as patient breathing, electrically charged electrodes, patient movement, or powerline interference. Baseline wander is undesirable as it can obscure useful and/or desirable information. Accordingly, the art has developed a number of techniques to mitigate baseline wander in a physiological waveform. These baseline mitigation techniques are sometimes referred to as "autoblocking".

SUMMARY

In a first aspect, a method for autoblocking a physiological waveform display to mitigate baseline wandering, comprises: extracting a low resolution preliminary baseline estimate from an input physiological waveform; extracting a high resolution deviation mean from the input physiological waveform; subtracting the low resolution preliminary baseline estimate from the high resolution deviation mean to extract a mean deviation of the input physiological waveform; determining a weight factor positively correlated to the mean deviation; applying the weight factor to the mean deviation of the input physiological waveform; applying the difference between 1 and the weight factor to the preliminary baseline estimate; combining the weighted mean deviation of the input physiological waveform with the weighted preliminary baseline estimate to extract an adjusted baseline estimate; and subtracting the adjusted baseline estimate from the input physiological waveform to obtain an autoblocked physiological waveform for display.

In a second aspect, a method for autoblocking a physiological waveform display to mitigate baseline wandering, comprises: extracting a low resolution preliminary baseline estimate from an input physiological waveform; extracting a high resolution deviation mean from the input physiological waveform; subtracting the preliminary baseline estimate from the deviation mean to extract a mean deviation of the input physiological waveform; determine a mean weight factor positively correlated to the mean deviation by obtaining the square of the mean deviation divided by a sum of the mean deviation and a constant; extracting an adjusted baseline estimate by summing the mean deviation weighted with the mean weight factor and the preliminary baseline estimate weighted with 1 minus the mean weight factor; subtracting the adjusted baseline estimate from the input physiological waveform to obtain the an autoblocked physiological waveform for display; subtracting the input physiological waveform from the high resolution deviation mean to extract a high resolution deviation; determining a deviation suppression weight factor by utilizing the mean weight factor and the high resolution deviation with a suppression coefficient; and applying the deviation suppression weight factor to the autoblocked physiological waveform; summing the weighted autoblocked physiological waveform and the adjusted baseline estimate to obtain a deviation suppressed corrected signal; and feeding the deviation suppressed corrected signal back to the low resolution preliminary baseline estimate generation.

In a third aspect, a method for autoblocking a physiological waveform display to mitigate baseline wandering, comprises: determining a high resolution mean of an input physiological waveform; determining a preliminary baseline estimation; generating a plurality of weights from the difference between the high resolution mean and the input physiological waveform and from the difference between the high resolution mean and the preliminary baseline estimate; weighting the high resolution mean with a first one of the plurality of weights and the preliminary baseline estimation with the difference between 1 and the first one of the plurality of weights; summing the weighted preliminary baseline estimate and the weighted high resolution mean and subtracting the input physiological waveform to obtain an autoblocked physiological waveform; weighting the autoblocked physiological waveform with a second one of the plurality of weights; and feeding the weighted autoblocked physiological waveform back to the preliminary baseline estimation.

In a fourth aspect, a physiological waveform monitor, comprises: a deviation estimating module that, in operation, generates a high resolution mean from an input physiological waveform; a preliminary baseline estimation module that, in operation, generates a preliminary baseline estimation; a weight generating module that, in operation, generates a plurality of weights based on signal magnitude from a deviation in the input physiological waveform and a mean deviation in the preliminary baseline estimation; a signal correction module, a display that, in operation, displays the autoblocked physiological waveform; and a feedback for the baseline corrected signal to the preliminary baseline estimation module. In operation, the signal correction module generates: an autoblocked physiological waveform from the difference between the input physiological waveform and a final estimated baseline, the final estimated baseline being the combination of the high resolution mean weighted with a first one of the plurality of weights and the preliminary baseline weighted with 1 minus the first one of the plurality of weights; and a baseline corrected signal from the combination of the autoblocked physiological waveform weighted with a second one of the plurality of weights and the final estimated baseline;

In a fifth aspect, a method for autoblocking a physiological waveform display to mitigate baseline wandering in a physiological waveform substantially as shown and described herein.

In a sixth aspect, a physiological monitoring device configured to mitigate baseline wandering in a physiological waveform substantially as shown and described herein.

In a seventh aspect, a local physiological system configured to mitigate baseline wandering in a physiological waveform substantially as shown and described herein.

In an eighth aspect, a larger physiological system configured to mitigate baseline wandering in a physiological waveform substantially as shown and described herein.

The above presents a simplified summary of the invention as claimed below in order to provide a basic understanding of some aspects of the invention. This summary is not an exhaustive overview of the invention. It is not intended to identify key or critical elements of the invention or to delineate the scope of the invention. Its sole purpose is to present some concepts in a simplified form as a prelude to the more detailed description that is discussed later.

BRIEF DESCRIPTION OF THE DRAWINGS

In the drawings, like reference numbers generally indicate identical, functionally similar, and/or structurally similar elements.

FIG. 1 presents a patient monitoring system according to one or more examples.

FIG. 2 illustrates a method for use in processing a physiological waveform representing a physiological parameter of a monitored patient in accordance with one or more embodiments.

FIG. 3 schematically depicts one particular implementation of a physiological monitoring device.

FIG. 4 illustrates a method in which the autoblocked physiological waveform produced by the method of FIG. 2 is used to adjust the preliminary baseline adjustment in the method of FIG. 2.

FIG. 5A and FIG. 5B illustrate another particular embodiment of method for autoblocking a physiological waveform display to mitigate baseline wandering in a physiological waveform.

FIG. 6 is a schematic, system block diagram depicting a system such as may be used to implement the functionality described relative to FIG. 1-FIG. 5.

FIG. 7 illustrates a method for fast autoblocking an input physiological waveform to mitigate baseline wandering in another embodiment.

FIG. 8 illustrates the baseline reset responsiveness of one or more embodiments to a large baseline wander.

FIG. 9 compares signal quality/fidelity in one or more embodiments.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the spirit and scope of the appended claims.

DETAILED DESCRIPTION

Illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

In physiological parameter waveform display on a patient monitor, baseline wander mitigation (a/k/a, autoblocking or baseline restoration) can sometimes significantly compromise signal quality by causing distortion or artifact(s) if a fast response is desired. This may sometimes cause significant delay to recover from if good signal fidelity is required in a particular context. Additionally, if autoblocking is triggered by waveform clipping with display boundaries, design complexity may increase if various display areas with different position and scaling are distributed across multiple monitoring devices. For instance, display control synchronization among network devices will cost more effort in implementation, including interface protocol design. This may affect product robustness when dealing with important clinical events, such as defibrillation (regulatory compliance).

The presently disclosed technique provides fast autoblocking with real time baseline estimation. The presently disclosed technique provides automatic baseline wander detection and correction. The technique is a robust solution to mitigate baseline artifacts quickly without detecting waveform clipping, and an adaptive baseline estimator can significantly improve signal quality-e.g., high fidelity and low distortion. More particularly, the fast autoblocking with real time baseline estimator technique disclosed herein avoids complex physiological waveform clipping detection when triggering baseline correction. This will simplify the design (cost effective) and reduce potential control errors and/or unwanted/unpredictable behaviors in clinical use, making the technique more robust. This technique also produces better signal fidelity/quality and less distortion while a fast response is results based on clinical and regulatory needs, where a conventional approach will cause significantly delay correcting baseline if higher signal quality is required. The system and methods described herein provide an improvement over previous methods. More particularly, the system and methods described herein are configured to restore the baseline to normal level in about 0.5 to 1.2 seconds while also producing better signal quality with less distortion.

The fast autoblocking with real time baseline estimator contains adaptive weight baseline estimation and signal subtraction. The baseline estimation is based on two-scale (time resolution) signal processing, the low-resolution branch will extract low frequency component for a preliminary baseline estimate. The high-resolution branch will extract high frequency component (*e.g.*, clinical features or artifacts) by subtracting the preliminary estimate from the original input signal. The high frequency variation signal should be suppressed to reduce its impact on baseline estimate, this can be achieved by applying a weight factor to the deviation (when exceeding a threshold), and the weighted deviation is utilized to correct the preliminary baseline estimate, which will help improve the accuracy of baseline estimator. The signal subtraction will subtract the estimated baseline from the original input signal and generate a corrected output (baseline reset to zero level). Considering a fixed length time window or finite impulse filter ("FIR") is employed, the maximum delay of the fast autoblocking can be well controlled (1 to 1.5 seconds) and the signal quality/fidelity is ensured due to accurate baseline estimation.

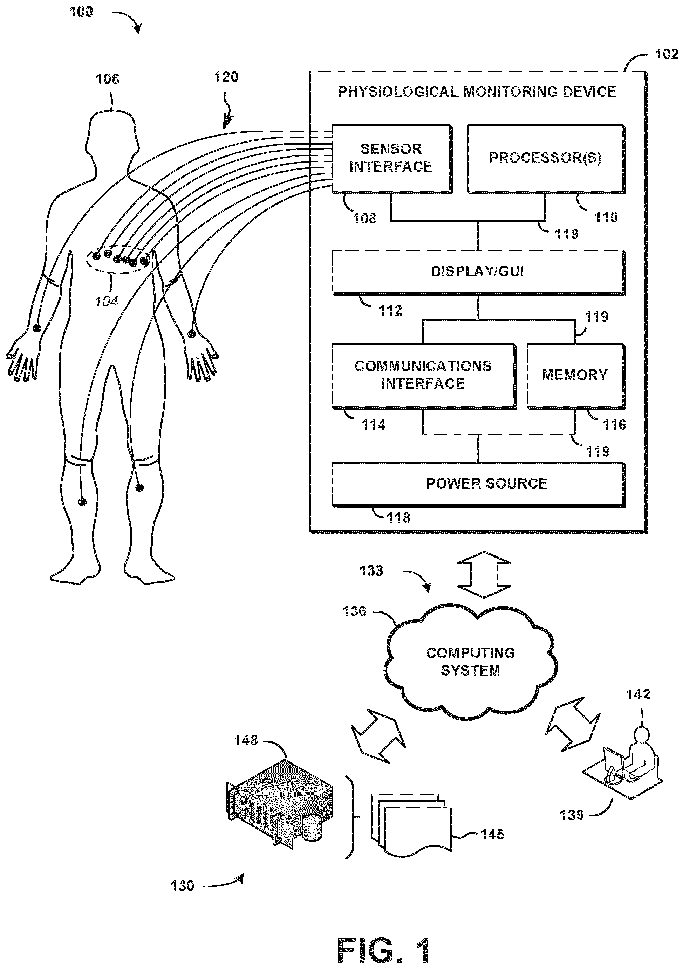

The presently disclosed technique is deployed in the context of monitoring a patient's physiological condition. Turning now to the drawings, FIG. 1 illustrates a local physiological monitoring system 100 according to one or more embodiments. As shown in FIG. 1, the system 100 includes a physiological monitoring device 102 capable of receiving physiological data from various sensors 104 connected to a patient 106 when deployed. In this example, the plurality of sensors 104 comprise electrocardiogram ("ECG") electrodes affixed to the skin of patient 106.

In general, it is contemplated by the present disclosure that physiological monitoring device 102 includes electronic components and/or electronic computing devices operable to receive, transmit, process, store, and/or manage patient physiological data and information associated with performing the functions of the system as described herein. These electronic components and/or electronic computing devices encompass any suitable processing device adapted to perform computing tasks consistent with the execution of computer-readable instructions stored in a memory or a computer-readable recording medium.

Further, any, all, or some of the computing devices in physiological monitoring device 102 may be adapted to execute any operating system, including Linux®, UNIX®, Windows Server®, etc., as well as virtual machines adapted to virtualize execution of a particular operating system, including customized and proprietary operating systems. The physiological monitoring device 102 may be further equipped with components to facilitate communication with other computing devices over one or more network connections, which may include connections to local and wide area networks, wireless and wired networks, public and private networks, and any other communication network enabling communication in the system.

As shown in FIG. 1, physiological monitoring device 102 may be, for example, a patient monitor implemented to monitor various physiological parameters of the patient 106 via the sensors 104. The physiological monitoring device 102 may include a sensor interface 108, one or more processors 110, a display/graphical user interface ("GUI") 112, a communications interface 114, a memory 116, and a power source (or power connection) 118, all communicating over an internal bus 119. The sensor interface 108 may be implemented in hardware or combination of hardware and software and is used to connect via wired and/or wireless connections to the sensors 104 for gathering physiological data from the patient 106. As noted, the sensors 104 in the present example are ECG electrodes affixed to the skin of the patient 106. A plurality of conductive leads 120, comprising a plurality of conductive cables, are provided for coupling the sensors 104 to the sensor interface 108. In one or more examples, the conductive leads 120 comprise a plurality of ECG cables.

The data signals from the sensors 104 may include, for example, sensor data related to an ECG. The one or more processors 110 may be used for controlling the general operations of the physiological monitoring device 102, as well as processing sensor data received by sensor interface 108. The one or more processors 110 may be any suitable processor-based resource. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of physiological monitoring device 102. In some embodiments, the one or more processors 110 may comprise a processor chipset including, for example and without limitation, one or more co-processors.

The display/GUI 112 may be configured to display various patient data, sensor data, and hospital or patient care information, and includes a user interface implemented for allowing interaction and communication between a user and physiological monitoring device 102. The display/GUI 112 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 112 may provide a means for inputting instructions or information directly to the physiological monitoring device 102. The patient information displayed may, for example, relate to the measured physiological parameters of the patient 106 (e.g., ECG readings).

The communications interface 114 may permit the physiological monitoring device 102 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 114 may include various network cards, interfaces, communication channels, cloud, antennas, and/or circuitry to permit wired and wireless communications with such computing networks and devices. The communications interface 114 may be used to implement, for example, a BLUETOOTH® connection, a cellular network connection, and/or a WIFI® connection with such computing networks and devices. Example wireless communication connections implemented using the communication interface 114 include wireless connections that operate in accordance with, but are not limited to, IEEE802.11 protocol, a Radio Frequency For Consumer Electronics ("RF4CE") protocol, and/or IEEE802.15.4 protocol (e.g., ZigBee® protocol). In essence, any wireless communication protocol may be used.

Additionally, the communications interface 114 may permit direct (i.e., device-to-device) communications (e.g., messaging, signal exchange, etc.) such as from a monitor mount to physiological monitoring device 102 using, for example, a universal serial bus ("USB") connection or other communication protocol interface. The communication interface 114 may also permit direct device-to-device connection to other devices such as to a tablet, computer, or similar electronic device; or to an external storage device or memory.

The memory 116 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 116 may be on-chip or off-chip depending on the implementation of the one or more processors 110. The memory 116 may be used to store any type of instructions and patient data associated with algorithms, processes, or operations for controlling the general functions and operations of the physiological monitoring device 102.

The power source 118 may include a self-contained power source such as a battery pack and/or include an interface to be powered through an electrical outlet, either directly or by way of a monitor mount. The power source 118 may also be a rechargeable battery that can be detached allowing for replacement. In the case of a rechargeable battery, a small built-in back-up battery (or super capacitor) can be provided for continuous power to be provided to the physiological monitoring device 102 during battery replacement. Communication between the components of the physiological monitoring device 102 in this example (may be established using the internal bus 119.

The physiological monitoring device 102 may be attached to one or more of several different types of sensors 104 and may be configured to measure and readout physiological data related to patient 106. As noted, the sensors 104 may be attached to the physiological monitoring device 102 by the conductive leads 120 which may be, for example, cables coupled to sensor interface 108. Additionally, or alternatively, one or more sensors 104 may connected to sensor interface 108 via a wireless connection. In which case sensor interface 108 may include circuity for receiving data from and sending data to one or more devices using, for example, a WIFI® connection, a cellular network connection, and/or a BLUETOOTH® connection.

The data signals received from the sensors 104, may be analog signals. For example, the data signals for the ECG may be input to the sensor interface 108, which can include an ECG data acquisition circuit (not shown separately in FIG. 1). An ECG data acquisition circuit may include amplifying and filtering circuity as well as analog-to-digital (A/D) circuity that converts the analog signal to a digital signal using amplification, filtering, and A/D conversion methods. In the event that the ECG sensor is a wireless sensor, the sensor interface 108 may receive the data signals from a wireless communication module (not shown in FIG. 1). Thus, the sensor interface 108 is a component which may be configured to interface with the one or more sensors 104 and receive sensor data therefrom.

As further described herein, the processing performed by physiological monitoring device 102 may generate analog data waveforms or digital data waveforms that are processed by, in this particular embodiment, a microcontroller. However, other embodiments may use other kinds of processors disclosed above. The microcontroller may be one of the processors 110.

The one or more processors 110, for example, may analyze the waveforms to identify certain waveform characteristics and threshold levels indicative of conditions (abnormal and normal) of the patient 106 using one or more monitoring methods. A monitoring method may include comparing an analog or a digital waveform characteristic or an analog or digital value to one or more threshold values and generating a comparison result based thereon. The microcontroller may be, for example, a processor, an FPGA, an ASIC, a DSP, a microcontroller, or similar processing device.

The microcontroller may include a memory (an on-chip memory) or use a separate memory 116 (an off-chip memory). The memory may be, for example, a RAM, a memory buffer, a hard drive, a database, an EPROM, an EEPROM, a ROM, a flash memory, a hard disk, or any other non-transitory computer readable medium. The memory 116 may store software or algorithms with executable instructions and the microcontroller may execute a set of instructions of the software or algorithms in association with executing different operations and functions of the physiological monitoring device 102 such as analyzing the digital data waveforms related to the data signals from the sensors 104.

As alluded to above, the physiological monitoring system 100 may also be deployed within the context of a larger patient monitoring system 130, also shown in FIG. 1, in some embodiments. The larger patient monitoring system 130 includes a computing system 133. The computing system 133 may be a distributed computing environment including a network or cloud 136 over which the physiological monitoring system 100 pushes and/or pulls data and information. The communications among the physiological monitoring device 102, the computing system 136, and other resources of the patient monitoring system 130 may either wireless or wired depending upon the technical capabilities of the various components.

For example, the facility (not otherwise shown) in which the patient 106 is located may include a central monitoring station 139 from which a caregiver 142 may monitor the physical condition of multiple patients 106 concurrently. The physiological monitoring device 102, using, for example, the communications interface 114 shown in FIG. 1, may push sensed physiological parameters and/or signals representative thereof to the central monitoring station 139 over the computing system 136. The pushed data and information may then be displayed for the caregiver 142 for review and consideration.

For another example, the physiological monitoring device 100 may pull information from an electronic medical record 145 for the patient 106 from a records repository 148 using, for example, the communications interface 114 shown in FIG. 1. The pulled information can then be displayed by the physiological monitoring device 102. Such pulled information might include, depending on the implementation and without limitation, medication regimens, diagnosed medical conditions, medical history, historical medical data, etc. Similarly, data sensed by or entered at the physiological monitoring device 102 may be pushed to, for instance, the ERM 145 for storage and later retrieval. Note, however, that in some embodiments the patient monitoring system 100 may not be integrated into or communicate with a larger patient monitoring system such as the larger patient monitoring system 130.

Accordingly, and still referring to FIG. 1, the presently disclosed technique processes the physiological data acquired from the sensors 104 from the patient 106 through the sensor interface 108 for display on the display/GUI 112. The processing and display occurs in "real-time" or "near real-time". As used herein, the terms "real-time" and "near real-time" means as nearly contemporaneously with the acquisition as can be technologically achieved and within time frames acceptable for clinical practice.

The technique may be implemented in either hardware or software, The embodiments disclosed herein perform the processing in software, with the one or more processor(s) 110 executing software instructions stored in, for example, the memory 116. Note that the acquired physiological data may be buffered and/or stored in the memory 116 as well. Although the illustrated embodiments perform the method in executable software, it is to be understood that other embodiments might perform the processing in hardware or some combination of hardware and software.

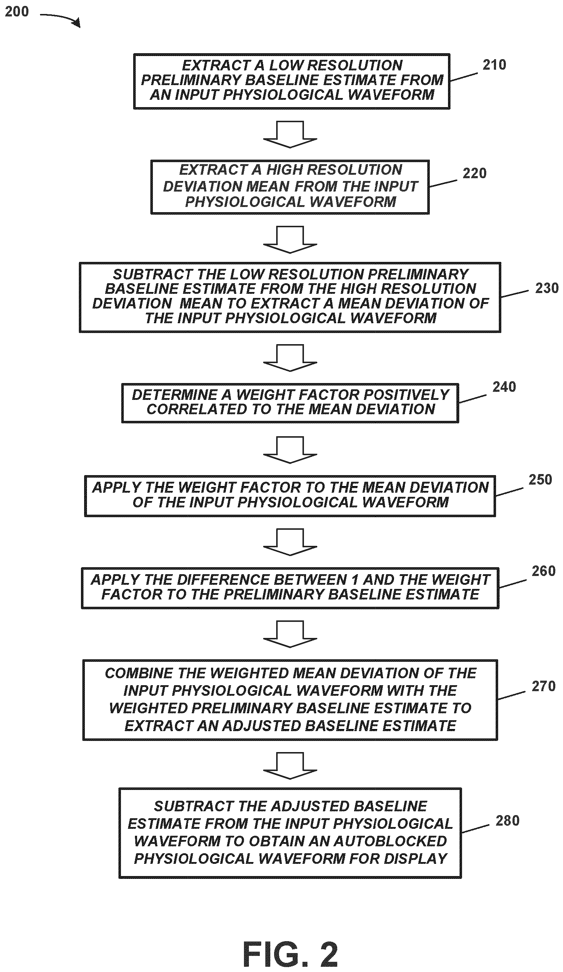

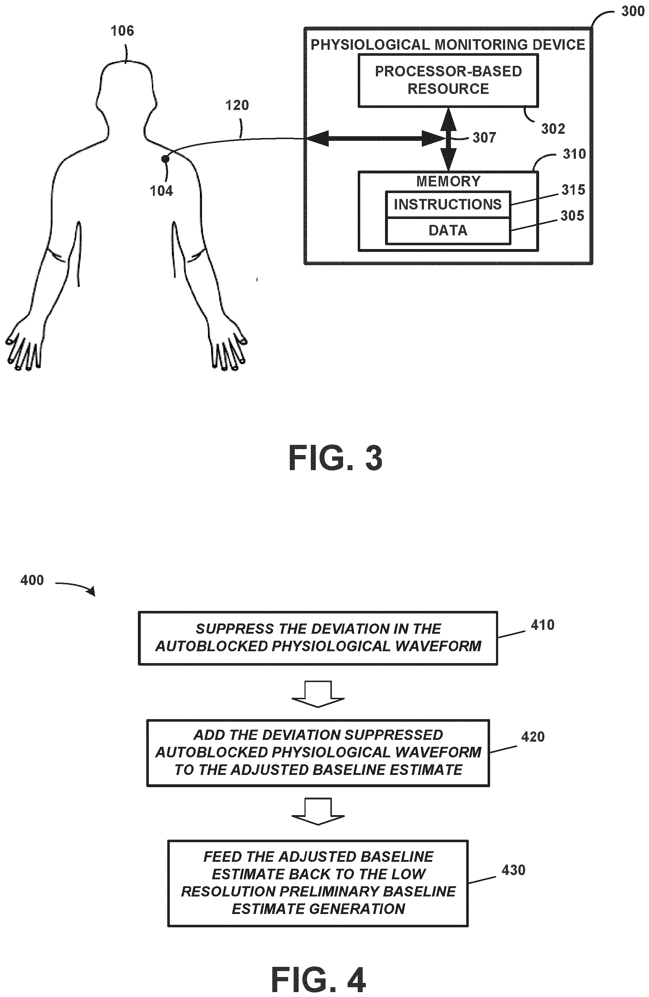

FIG. 2 illustrates a method 200 for use in processing a physiological waveform representing a physiological parameter of a monitored patient in accordance with one or more embodiments. FIG. 3 schematically depicts one particular implementation of a physiological monitoring device 300. The depiction of the physiological monitoring device 300 is simplified from what is shown in FIG. 1 so as not to obscure those aspects of the claimed subject matter now under discussion. More particularly, as illustrated in FIG. 3, a processor-based resource 302 communicates with a memory 310 over an internal bus system 307. Thus, some of the details discussed relative to and shown in FIG. 1 have been omitted for purposes of this discussion although such details will be pertinent in most embodiments.

The processor-based resource 302 may be, for example, one of the one or more processors 110 shown in FIG. 1 and is implemented, in this particular embodiment, in a microcontroller. The processor-based resource 302 operates under programmed control of the instructions 315. The processor-based resource 302 therefore communicates with the memory 310 over the bus system 307 to, among other things, retrieve and execute the instructions 315 stored therein.

The execution of the instructions may cause the processor-based resource 302 to perform certain tasks such as some portions of the method 200 outlined in FIG. 2. However, depending on the environment, the processor-based resource 302 may also perform other tasks such as pushing data to other computing resources, transmitting alarms, *etc.* Thus, in this sense, the processor-based resources 302, as well as the physiological monitoring device 300 as a whole, may be considered to be "programmed" or "configured" to perform certain functions as described herein.

Referring now collectively to FIG. 2 and FIG. 3, the method 200 assumes that the physiological data has been acquired. Acquisition includes acquiring a time domain signal representative of the physiological parameter. This time domain signal is acquired via the selected lead 120 and sensor 104 as described above. Note that the sensor 104 is representative and that other sensors (not shown in FIG. 3) may be used in other embodiments. In this particular embodiment, the sensor 104 is an ECG sensor and is one of a plurality of ECG sensors communicating with the physiological monitoring device 300. The acquired time domain signal is transmitted from the sensor 104 to the physiological monitoring device 300 over the lead 120.

The acquired time domain signal as received by the physiological monitoring device 300 may be in either analog or digital form. If received in analog form, it is converted to digital. Once digitized, the resulting data is buffered as data 305 in the memory 310. The memory 310 in this embodiment is off-chip and includes both RAM and ROM in both volatile and non-volatile memory. The data 305 may be buffered or stored in any suitable kind of data structure.

Note that the information derived from the acquired time domain signal will typically be displayed in real time or near real time in most embodiments. Thus, the data 305 representing the acquired time domain signal may be temporarily stored, or buffered, in the memory 310. Some embodiments may, however, store the data 305 representing the acquired time domain signal in portions of memory 310 more suitable for long term storage in addition to buffering. Some embodiments may even push such data 305 to other computing resources for longer term storage as discussed relative to FIG. 1.

The processing of the data 305 may be performed either in hardware (not shown) or in software by the processor-based resource 302. The illustrated embodiment performs this task in software. As the data 305 begins arriving, the processor-based resource 302 begins processing the data 305 by retrieving and executing the instructions 315 to perform the method 200 in FIG. 2. The processor-based resource 302 may therefore be said to be "programmed" or "configured to" perform the method 200. Note that, depending on the particular embodiment, the processor-based resource 302 may process the data 305 immediately upon receipt or may retrieve it from a buffer in the memory 310.

The method 200 begins by extracting (at 210) a low resolution preliminary baseline estimate and extracting (at 220) a high resolution deviation mean from an input physiological waveform. The input physiological waveform is an acquired physiological waveform or a baseline corrected signal. In general, if a baseline corrected signal is available, the baseline corrected signal is preferred over the acquired physiological waveform.

The method 200 then subtracts (at 230) the low resolution preliminary baseline estimate from the high resolution deviation mean. This extracts a mean deviation of the input physiological waveform. The mean deviation is then used to determine (at 240) a weight factor positively correlated to the mean deviation. In the illustrated embodiments, the weight factor is between 0 and 1. In one particular embodiment, determining (at 240) a weight factor positively correlated to the mean deviation includes obtaining the square of the mean deviation divided by sum of mean deviation and a constant. The constant may be, for example, $1\pm10\%$.

Next, the method applies (at 250) the weight factor to the mean deviation of the input physiological waveform and applies (at 260) the complement, i.e., the difference between 1 and the weight factor to the preliminary baseline estimate. Note that these steps may be performed in either order.

The method 200 continues by combining (at 270) the weighted mean deviation of the input physiological waveform with the weighted preliminary baseline estimate to extract an adjusted baseline estimate. This act suppresses the deviation in the preliminary baseline estimate. Then, the method 200 subtracts (at 280) the adjusted baseline estimate from the input physiological waveform to obtain an autoblocked physiological waveform for display. The autoblocked physiological waveform may be displayed on, for example, the local display/GUI of the physiological monitoring device. Note that some embodiments may store the autoblocked physiological waveform longer term. Some embodiments may transmit the autoblocked physiological waveform to a remote location as discussed above for remote display.

In some embodiments the autoblocked physiological waveform may be used in generating the low resolution preliminary baseline estimate as illustrated in FIG. 4. This feedback 400 begins by suppressing (at 410) the deviation in the autoblocked physiological waveform. This may be done in some embodiments by first subtracting the input physiological waveform from the high resolution deviation mean to extract a high resolution deviation. Then, a deviation suppression weight factor is determined by utilizing the mean weight factor and the high resolution deviation with a suppression coefficient. The deviation suppression weight factor is then applied to the autoblocked physiological waveform. Then, returning to FIG. 4, the method 400 continues by adding (at 420) the deviation suppressed autoblocked physiological waveform to the adjusted baseline estimate; and feeding (at 430) the adjusted baseline estimate back to the low resolution preliminary baseline estimate generation.

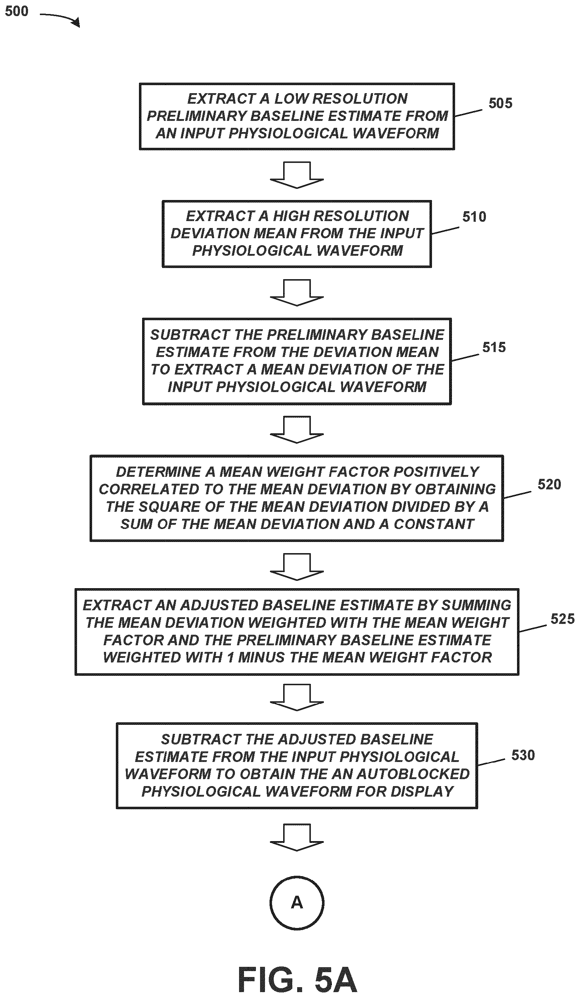

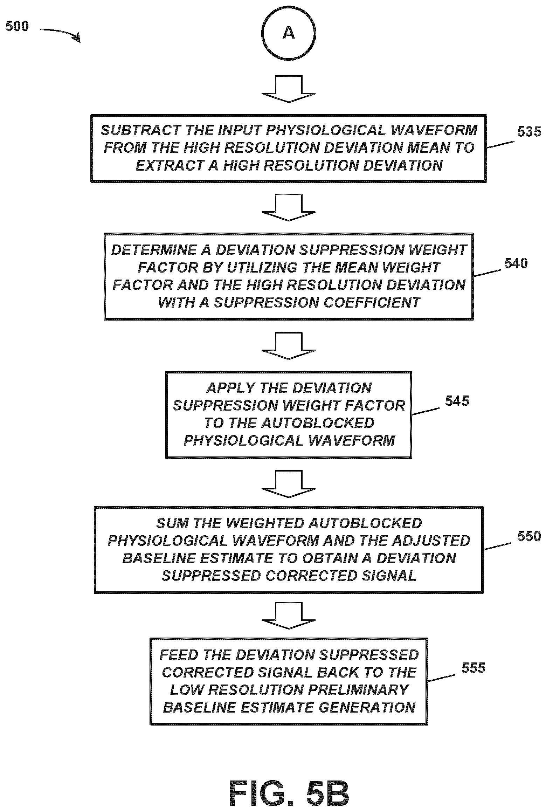

FIG. 5 illustrates another particular embodiment of method for autoblocking a physiological waveform display to mitigate baseline wandering in a physiological waveform. The method 500 can be performed on a physiological parameter monitor such as those shown in FIG. 1 and FIG. 3.

The method 500 begins by extracting (at 505) a low resolution preliminary baseline estimate from an input physiological waveform. The input physiological waveform is an acquired physiological waveform or a baseline corrected signal. In general, if a baseline corrected signal is available, the baseline corrected signal is preferred over the acquired physiological waveform. The method 500 then extracts (at 510) a high resolution deviation mean from the input physiological waveform;

Next, the method 500 subtracts (at 515) the preliminary baseline estimate from the deviation mean to extract a mean deviation of the input physiological waveform. This is followed by determining (at 520) a mean weight factor positively correlated to the mean deviation by obtaining the square of the mean deviation divided by a sum of the mean deviation and a constant. In the illustrated embodiments, the mean weight factor is between 0 and 1 and the constant is $1\pm10\%$.

The method 500 continues by extracting (at 525) an adjusted baseline estimate by summing the mean deviation weighted with the mean weight factor and the preliminary baseline estimate weighted with 1 minus the mean weight factor. Next, the method 500 subtracts (at 530) the adjusted baseline estimate from the input physiological waveform to obtain the an autoblocked physiological waveform for display. The adjusted baseline estimate tracks the baseline wander in a responsive manner.

The method 500 then proceeds to the optional feedback discussed above. The method 500 subtracts (at 535) the input physiological waveform from the high resolution deviation mean to extract a high resolution deviation. Next, a deviation suppression weight factor is determined (at 540) by utilizing the mean weight factor and the high resolution deviation with a suppression coefficient. In the illustrated embodiment, the deviation suppression weight factor is between 0 and 1 and the suppression coefficient is $10\pm10\%$.

Next, the method 500 applies (at 545) the deviation suppression weight factor to the autoblocked physiological waveform. The method 500 then sums (at 550) the weighted autoblocked physiological waveform and the adjusted baseline estimate to obtain a deviation suppressed corrected signal. The deviation suppressed corrected signal is then fed back (at 555) to the low resolution preliminary baseline estimate generation.

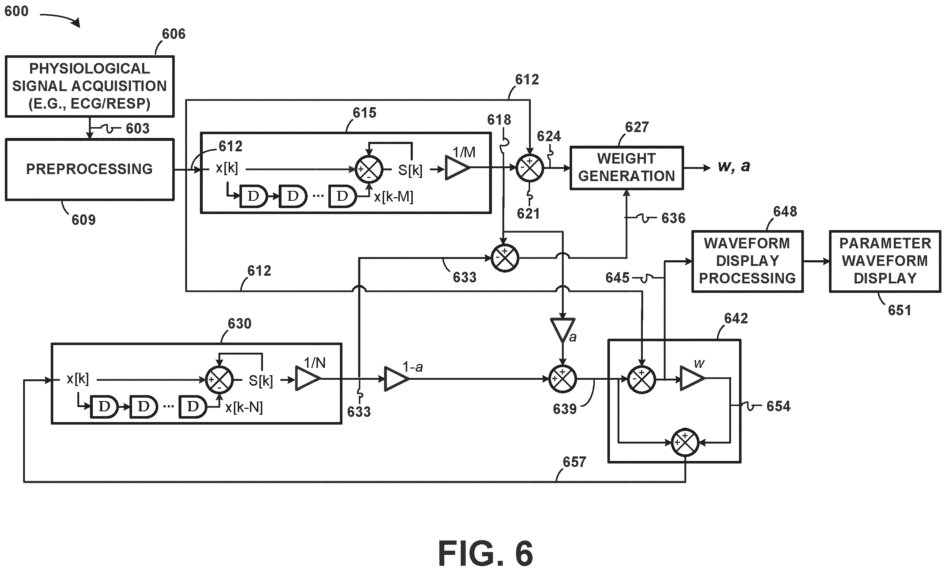

FIG. 6 is a schematic, system block diagram of a system 600 such as may be used to implement the functionality described above. The fast autoblocking technique disclosed herein is designed based on clinical and regulatory concerns to achieve quality display of physiological parameter waveform and mitigation of baseline wander artifacts in a responsive manner.

The raw physiological signals 603 are acquired by the front-end system 606. This may be performed as described above relative to FIG. 1 and FIG. 3. As noted above, the embodiments disclosed herein perform an ECG acquisition, but the technique disclosed herein is not so limited. In various embodiments the acquisition may be of, for example and without limitation, blood pressure, body temperature, vision acuity, quantities of various substances in the blood, etc. The actual acquisition 606 will therefore depend on the nature of the data being acquired and will be well known to the art. Accordingly, further discussion of the acquisition 606 will be omitted for the sake of clarity and so as not to obscured that which is claimed below.

The raw physiological signals 603 are then preprocessed with appropriate filters 609 to obtain desired signal format for the input signal-i.e., the input physiological waveform 612. As those in the art having the benefit of this disclosure will appreciate, the nature and extent of the preprocessing, and therefore the nature of the filters 609, will depend to some degree on the type of data being acquired. The precise nature of the processing not only depends on the nature of the data acquired, but is also routine and well known to the art. Accordingly, further discussion of the preprocessing will also be omitted for the sake of clarity and so as not to obscured that which is claimed below.

The input physiological waveform 612 is then input to the deviation estimation module 615. The deviation estimate module 615 employs a high resolution, moving average finite impulse response ("FIR") filter with a small window size. In the deviation estimation module 615 in FIG. 6, D is a unity delay and M is the moving average window size. The illustrated embodiment uses a 0.2 second window size for typical physiological signals. The FIR filter generates a high resolution mean 618 of the input physiological waveform 612. The high resolution mean 618 is then subtracted from the input physiological waveform 612 at the point 621 to obtain a signal deviation 624. The signal deviation 624 yields clinical features or sharp artifacts.

The signal deviation 624 is then used in the weight generation module 627 to generate weight factors w and a. The determination of the weight factors w, a in this particular embodiment begins with the mean deviation, which can be represented as $D_m = |M_h - B_0|$, where $M_h$ is the high resolution mean, $B_0$ is the preliminary baseline estimate. The weight factor to compute estimate baseline $a = (\frac{D_m}{D_m + C})^2$, where c is unit signal magnitude (e.g., 1mV). Given signal deviation d = $x - M_h$ (where x is the input physiological waveform), the weight factor may be represented as $w = a + \frac{1-a}{1+K_w \cdot d}$, where $K_w$ is a deviation suppression threshold coefficient in range of 10+/-1. If signal deviation increases, the weight factor w will decrease to suppress the impact of deviation.

The preliminary baseline estimation module 630 employs a low resolution, moving average FIR with large window size. In the baseline estimation module 630, D is a unity delay and N is a moving average window size. In this context, the window size may be 1 to 2 seconds depending in the type of physiological signal to generate a low resolution mean of the corrected input physiological waveform. For example, 1.2 seconds is appropriate for an ECG waveform. The preliminary baseline estimation module 630 generates the preliminary baseline estimation 633.

To track baseline wander in a responsive manner, a weight factor (a≤1) is derived based on the mean deviation 636, which is the difference between the high resolution mean 618 and preliminary baseline estimate 633. The mean deviation 636 is positively correlated to a high resolution mean 618 to track fast baseline change. The weight factor is applied to a linear combination of preliminary baseline estimate 633 and high resolution mean 618, which is the final estimated baseline 639. The final estimated baseline 639 is subtracted from the input physiological waveform 612 in the signal correction module 642 to generate the output signal, i.e., the autoblocked physiological waveform 645. The autoblocked physiological waveform 645 then undergoes waveform display processing 648 for parameter waveform display 651.

Still referring to the signal correction module 642, to mitigate the impact of high physiological features and high magnitude artifacts on the baseline estimate, a suppression weight factor w (w≤1 ) is derived based on signal deviation and a weight factor a. The factor w is inversely correlated to signal deviation and applied to the input physiological waveform 612 to suppress high resolution deviation. The weighted autoblocked physiological waveform 654 is then combined with weighted estimated baseline to generate a corrected signal 657. The corrected signal 657 is then fed into the baseline estimation module 630 as shown in FIG. 6. Note that, as mentioned above, the signal correction in the signal correction module 642 is optional and may not be performed in all embodiments.

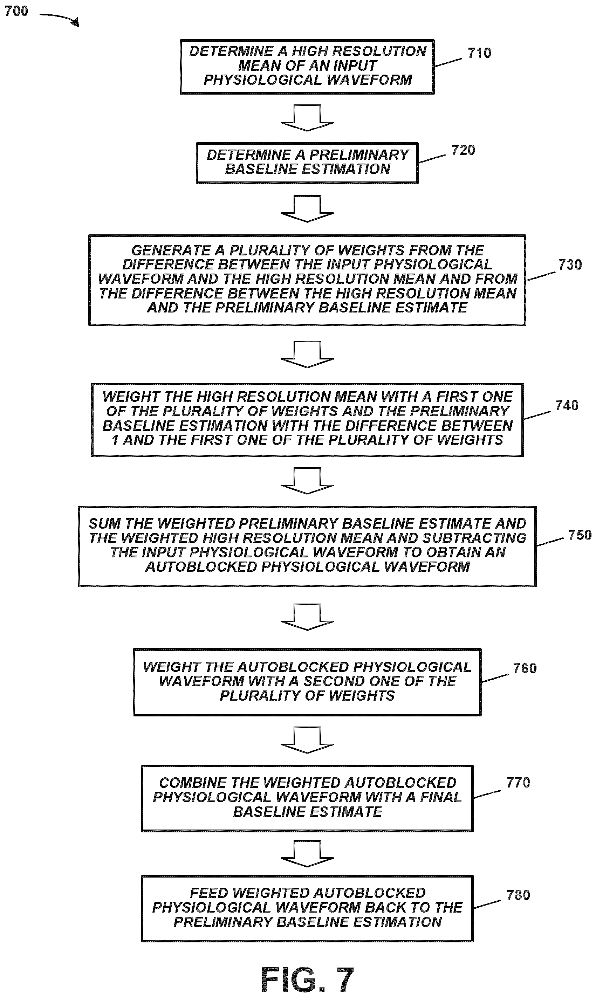

FIG. 7 illustrates a method 700 for fast autoblocking an input physiological waveform to mitigate baseline wandering in another embodiment. Referring to FIG. 6 and FIG. 7 collectively, the method 700 begins with determining (at 710) a high resolution mean 618 of an input physiological waveform 612. The input physiological waveform 612 is an acquired physiological waveform or a baseline corrected signal. In general, if a baseline corrected signal is available, the baseline corrected signal is preferred over the acquired physiological waveform. The method 700 then determines (at 720) a preliminary baseline estimation 633.

Next, the method 700 generates (at 730) a plurality of weights w, a from the difference 624 between the input physiological waveform 612 and the high resolution mean 618 and from the difference 633 between the high resolution mean 618 and the preliminary baseline estimate 633. The difference 624 between the high resolution mean 618 and the input physiological waveform 612 represents a signal deviation. The difference 624 between the high resolution mean 618 and the preliminary baseline estimate 633 represents a mean deviation.

The method 700 then weights (at 740) the high resolution mean 618 with a first one of the plurality of weights and the preliminary baseline estimation with the difference between 1 and the first one of the plurality of weights a. The weighted preliminary baseline estimate and the weighted high resolution mean are then summed (at 750) and the input physiological waveform subtracted from the sum to obtain an autoblocked physiological waveform 645. The autoblocked physiological waveform 645 is also weighted (at 760) with a second one of the plurality of weights w. The weighted autoblocked physiological waveform 654 is then combined (770) with a final baseline estimation 639 and the combination 657 fed back (at 780) to the preliminary baseline estimation.

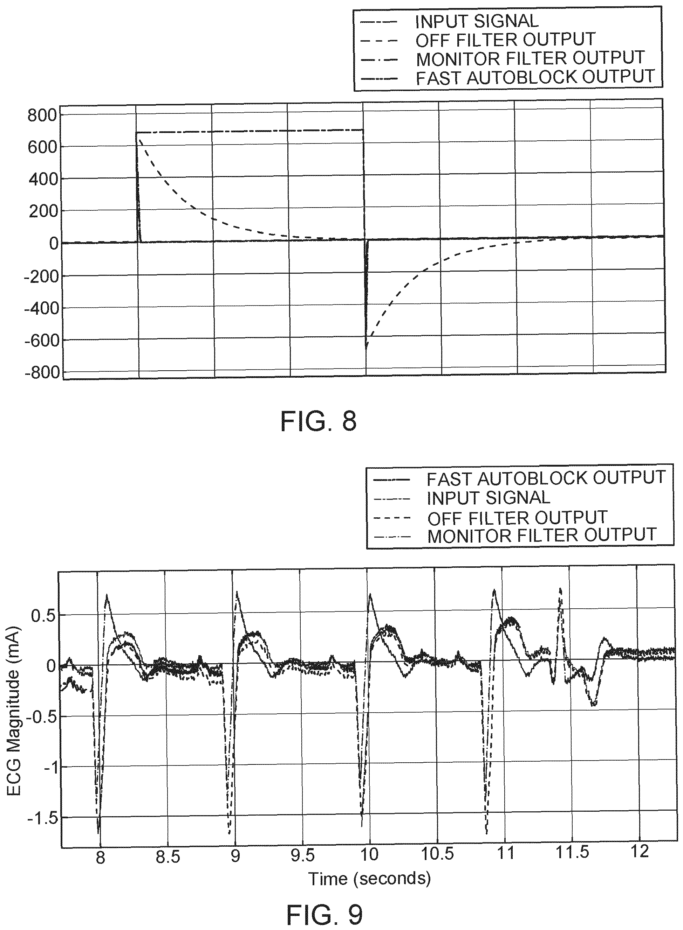

FIG. 8 demonstrates the time response of different approaches to large baseline wander (e.g., caused by a defib signal in an ECG signal). The fast autoblocking and monitor technique disclosed herein (IIR with 0.67Hz cutoff frequency) will restore the baseline to normal level in about 0.5 to 1.2 second (in red) while an Off Filter (in green) will take 15-20 seconds.

A comparison of signal quality/fidelity is illustrated in FIG. 9. The fast autoblocking (red) and Off Filter (green) demonstrate good signal fidelity compared to the input physiological signal (yellow). There is no attenuation and distortion observed. The Monitor Filter (light blue) shows noticeable signal attenuation and distortion, which is not suitable for clinical use cases requiring higher signal quality (e.g., ST segment analysis).

Accordingly, in a first embodiment, a method for autoblocking a physiological waveform display to mitigate baseline wandering, comprises: extracting a low resolution preliminary baseline estimate from an input physiological waveform; extracting a high resolution deviation mean from the input physiological waveform; subtracting the low resolution preliminary baseline estimate from the high resolution deviation mean to extract a mean deviation of the input physiological waveform; determining a weight factor positively correlated to the mean deviation; applying the weight factor to the mean deviation of the input physiological waveform; applying the difference between 1 and the weight factor to the preliminary baseline estimate; combining the weighted mean deviation of the input physiological waveform with the weighted preliminary baseline estimate to extract an adjusted baseline estimate; and subtracting the adjusted baseline estimate from the input physiological waveform to obtain an autoblocked physiological waveform for display.

In a second embodiment, the input physiological waveform in the first embodiment is an acquired physiological waveform or a baseline corrected signal.

In a third embodiment, the weight factor in the first embodiment is between 0 and 1.

In a fourth embodiment, determining a weight factor positively correlated to the mean deviation in the first embodiment includes obtaining the square of the mean deviation divided by sum of mean deviation and a constant.

In a fifth embodiment, the constant in the first embodiment is $1 \pm 10\%$.

In a sixth embodiment, combining the weighted mean deviation of the input physiological waveform with the weighted preliminary baseline estimate in the first embodiment suppresses the deviation in the preliminary baseline estimate.

In a seventh embodiment, the method the first embodiment further comprises: suppressing the deviation in the autoblocked physiological waveform; adding the deviation suppressed autoblocked physiological waveform to the adjusted baseline estimate; and feeding the adjusted baseline estimate back to the low resolution preliminary baseline estimate generation.

In an eighth embodiment suppressing the deviation in the autoblocked physiological waveform in the first embodiment further comprises: subtracting the input physiological waveform from the high resolution deviation mean to extract a high resolution deviation; determining a deviation suppression weight factor by utilizing the mean weight factor and the high resolution deviation with a suppression coefficient; and applying the deviation suppression weight factor to the autoblocked physiological waveform.

In a ninth embodiment, the deviation suppression weight factor in the first embodiment is between 0 and 1.

In a tenth embodiment, the suppression coefficient in the seventh embodiment is $10 \pm 10\%$.

In an eleventh embodiment, a method for autoblocking a physiological waveform display to mitigate baseline wandering, comprises: extracting a low resolution preliminary baseline estimate from an input physiological waveform; extracting a high resolution deviation mean from the input physiological waveform; subtracting the preliminary baseline estimate from the deviation mean to extract a mean deviation of the input physiological waveform; determine a mean weight factor positively correlated to the mean deviation by obtaining the square of the mean deviation divided by a sum of the mean deviation and a constant; extracting an adjusted baseline estimate by summing the mean deviation weighted with the mean weight factor and the preliminary baseline estimate weighted with 1 minus the mean weight factor; subtracting the adjusted baseline estimate from the input physiological waveform to obtain the an autoblocked physiological waveform for display; subtracting the input physiological waveform from the high resolution deviation mean to extract a high resolution deviation; determining a deviation suppression weight factor by utilizing the mean weight factor and the high resolution deviation with a suppression coefficient; and applying the deviation suppression weight factor to the autoblocked physiological waveform; summing the weighted autoblocked physiological waveform and the adjusted baseline estimate to obtain a deviation suppressed corrected signal; and feeding the deviation suppressed corrected signal back to the low resolution preliminary baseline estimate generation.

In a twelfth embodiment, the input physiological waveform in the eleventh embodiment is a raw, acquired waveform or a baseline corrected signal.

In a thirteenth embodiment, the mean weight factor in the eleventh embodiment is between 0 and 1.

In a fourteenth embodiment, the constant in the eleventh embodiment is $1\pm10\%$.

In a fifteenth embodiment, the adjusted baseline estimate in the eleventh embodiment tracks the baseline wander in a responsive manner.

In a sixteenth embodiment, the deviation suppression weight factor in the eleventh embodiment is between 0 and 1.

In a seventeenth embodiment, the suppression coefficient in the eleventh embodiment is $10\pm10\%$.

In an eighteenth embodiment, the eleventh embodiment further comprises displaying the autoblocked physiological waveform.

In a nineteenth embodiment, a method for autoblocking a physiological waveform display to mitigate baseline wandering, comprises: determining a high resolution mean of an input physiological waveform; determining a preliminary baseline estimation; generating a plurality of weights from the difference between the high resolution mean and the input physiological waveform and from the difference between the high resolution mean and the preliminary baseline estimate; weighting the high resolution mean with a first one of the plurality of weights and the preliminary baseline estimation with the difference between 1 and the first one of the plurality of weights; summing the weighted preliminary baseline estimate and the weighted high resolution mean and subtracting the input physiological waveform to obtain an autoblocked physiological waveform; weighting the autoblocked physiological waveform with a second one of the plurality of weights; combining the weighted autoblocked physiological waveform with a final baseline estimate; and feeding the weighted autoblocked physiological waveform back to the preliminary baseline estimation.

In a twentieth embodiment, the input physiological waveform in the nineteenth embodiment is a raw, acquired waveform or a baseline corrected signal.

In a twenty-first embodiment, the difference between the high resolution mean and the input physiological waveform in the nineteenth embodiment represents a signal deviation.

In a twenty-second embodiment, the difference between the high resolution mean and the preliminary baseline estimate in the nineteenth embodiment represents a mean deviation.

In a twenty-third embodiment, summing the weighted preliminary baseline estimate and the weighted high resolution mean in the nineteenth embodiment yields a final baseline estimation.

In a twenty-fourth embodiment, a physiological waveform monitor, comprises: a deviation estimating module that, in operation, generates a high resolution mean from an input physiological waveform; a preliminary baseline estimation module that, in operation, generates a preliminary baseline estimation; a weight generating module that, in operation, generates a plurality of weights based on signal magnitude from a deviation in the input physiological waveform and a mean deviation in the preliminary baseline estimation; a signal correction module; and a display that, in operation, displays the autoblocked physiological waveform. The signal correction module, in operation, generates an autoblocked physiological waveform from the difference between the input physiological waveform and a final estimated baseline, the final estimated baseline being the combination of the high resolution mean weighted with a first one of the plurality of weights and the preliminary baseline weighted with 1 minus the first one of the plurality of weights; and a baseline corrected signal from the combination of the autoblocked physiological waveform weighted with a second one of the plurality of weights and the final estimated baseline; and a feedback for the baseline corrected signal to the preliminary baseline estimation module; and

In a twenty-fifth embodiment, the input physiological waveform in the twenty-fourth embodiment is a raw, acquired waveform or a baseline corrected signal.

In a twenty-sixth embodiment, the deviation estimation module in the twenty-fourth embodiment further comprises a high resolution, moving average finite impulse response ("FIR") filter with a small window size.

In a twenty-seventh embodiment, the small window size in the twenty-fourth embodiment is 0.2 seconds.

In a twenty-eighth embodiment, generating a high resolution mean from an input physiological waveform in the twenty-fourth embodiment includes applying a high resolution, moving average finite impulse response filter with a small window size to the input physiological waveform.

In a twenty-ninth embodiment, the small window size in the twenty-fourth embodiment is 0.2 seconds.

In a thirtieth embodiment, the deviation in the input physiological waveform in the twenty-fourth embodiment is the difference between the input physiological waveform and a high resolution mean of the input physiological waveform.

In a thirty-first embodiment, the preliminary baseline estimation module in the twenty-fourth embodiment further comprises a moving average finite impulse response filter with a large window size.

In a thirty-second embodiment, the large window size in the thirty-first embodiment is 1 to 2 seconds.

In a thirty-third embodiment, generating a preliminary baseline estimation in the twenty-fourth embodiment includes applying a moving average finite impulse response filter with a large window size to the correction signal.

In a thirty fourth embodiment the large window size in the thirty-third embodiment is 1 to 2 seconds.

In a thirty-fifth embodiment, a method for autoblocking a physiological waveform display to mitigate baseline wandering in a physiological waveform is substantially as shown and described.

In a thirty-sixth embodiment, a physiological monitoring device is configured to mitigate baseline wandering in a physiological waveform substantially as shown and described.

In a thirty seventh embodiment, a local physiological system is configured to mitigate baseline wandering in a physiological waveform substantially as shown and described.

In a thirty-eighth embodiment, a larger physiological system configured to mitigate baseline wandering in a physiological waveform is substantially as shown and described.

Expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the following, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the following description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

ECG signal processing, as used herein, refers to, without limitation, manipulating an analog signal in such a way that the signal meets the requirements of a next stage for further processing. ECG signal processing may include converting between analog and digital realms (e.g., via an analog-to-digital or digital-to-analog converter), amplification, filtering, converting, biasing, range matching, isolation and any other processes required to make a sensor output suitable for processing.

The term "module" is accorded herein its ordinary and accustomed meaning in the art-hardware, software, or a combination of hardware and software that implements some desired function. The interchangeability between software and hardware is well known in the art and finds applicability in technological contexts such as those disclosed herein. Thus, a module may be implemented in, for example, electronic circuits comprised of electronic components such as transistors, resistors, inductors, capacitor, etc. Or, a module may be implemented in software in the form of executable instructions stored by a processor-based resource. Or, a module may be implemented in an integrated circuit such as an ASIC, an EPROM, or an EEPROM. Or, perhaps, in some combination of these choices.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner.

Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold).

The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the spirit and scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the spirit and scope of the present disclosure.

Various modifications to the disclosure will therefore be readily apparent to those skilled in the art, and the generic principles defined herein may be applied to other variations without departing from the spirit or scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

The present disclosure includes the subject matter set out in the following numbered Statements:

Statement 1. A method for autoblocking a physiological waveform display to mitigate baseline wander, comprising:

extracting a low resolution preliminary baseline estimate from an input physiological waveform;

extracting a high resolution deviation mean from the input physiological waveform;

subtracting the low resolution preliminary baseline estimate from the high resolution deviation mean to extract a mean deviation of the input physiological waveform;

determining a weight factor positively correlated to the mean deviation;

applying the weight factor to the mean deviation of the input physiological waveform;

applying the difference between 1 and the weight factor to the preliminary baseline estimate;

combining the weighted mean deviation of the input physiological waveform with the weighted preliminary baseline estimate to extract an adjusted baseline estimate; and

subtracting the adjusted baseline estimate from the input physiological waveform to obtain an autoblocked physiological waveform for display.

Statement 2. The method of Statement 1, wherein the input physiological waveform is an acquired physiological waveform or a baseline corrected signal.

Statement 3. The method of Statement 1 or 2, wherein the weight factor is between 0 and 1.

Statement 4. The method of any of Statements 1-3, wherein determining a weight factor positively correlated to the mean deviation includes obtaining the square of the mean deviation divided by sum of mean deviation and a constant

Statement 5. The method of any of Statements 1-4, wherein the constant is $1\pm10\%$.

Statement 6. The method any of Statements 1-5, wherein combining the weighted mean deviation of the input physiological waveform with the weighted preliminary baseline estimate suppresses the deviation in the preliminary baseline estimate.

Statement 7. The method of any of Statements 1-6, further comprising:

suppressing the deviation in the autoblocked physiological waveform;

adding the deviation suppressed autoblocked physiological waveform to the adjusted baseline estimate; and

feeding the adjusted baseline estimate back to the low resolution preliminary baseline estimate generation.

Statement 8. The method of any of Statements 1-7, wherein suppressing the deviation in the autoblocked physiological waveform further comprises:

subtracting the input physiological waveform from the high resolution deviation mean to extract a high resolution deviation;

determining a deviation suppression weight factor by utilizing the mean weight factor and the high resolution deviation with a suppression coefficient; and

applying the deviation suppression weight factor to the autoblocked physiological waveform

Statement 9. The method of Statement 8, wherein the deviation suppression weight factor is between 0 and 1.

Statement 10. The method of Statement 8 or 9, wherein the suppression coefficient is $10\pm10\%$.

Statement 11. A method for autoblocking a physiological waveform display to mitigate baseline wander, comprising:

extracting a low resolution preliminary baseline estimate from an input physiological waveform;

extracting a high resolution deviation mean from the input physiological waveform;

subtracting the preliminary baseline estimate from the deviation mean to extract a mean deviation of the input physiological waveform;

determine a mean weight factor positively correlated to the mean deviation by obtaining the square of the mean deviation divided by a sum of the mean deviation and a constant;

extracting an adjusted baseline estimate by summing the mean deviation weighted with the mean weight factor and the preliminary baseline estimate weighted with 1 minus the mean weight factor;

subtracting the adjusted baseline estimate from the input physiological waveform to obtain an autoblocked physiological waveform for display;

subtracting the input physiological waveform from the high resolution deviation mean to extract a high resolution deviation;

determining a deviation suppression weight factor by utilizing the mean weight factor and the high resolution deviation with a suppression coefficient;

applying the deviation suppression weight factor to the autoblocked physiological waveform;

summing the weighted autoblocked physiological waveform and the adjusted baseline estimate to obtain a deviation suppressed corrected signal; and

feeding the deviation suppressed corrected signal back to the low resolution preliminary baseline estimate generation.

Statement 12. The method of Statement 11, wherein the input physiological waveform is a raw, acquired waveform or a baseline corrected signal.

Statement 13. The method of Statement 11 or 12, wherein the mean weight factor is between 0 and 1

Statement 14. The method of any of Statements 11-13, wherein the constant is $1\pm10\%$.

Statement 15. The method of any of Statements 11-14, wherein the adjusted baseline estimate tracks the baseline wander in a responsive manner.

Statement 16. The method of any of Statements 11-15, wherein the deviation suppression weight factor is between 0 and 1.

Statement 17. The method of any of Statements 11-16, wherein the suppression coefficient is 10 $\pm10\%$.

Statement 18. The method of any of Statements 11-17, further comprising displaying the autoblocked physiological waveform.

Statement 19. A method for autoblocking a physiological waveform display to mitigate baseline wander, comprising:

determining a high resolution mean of an input physiological waveform;

determining a preliminary baseline estimation;

generating a plurality of weights from the difference between the high resolution mean and the input physiological waveform and from the difference between the high resolution mean and the preliminary baseline estimate;

weighting the high resolution mean with a first one of the plurality of weights and the preliminary baseline estimation with the difference between 1 and the first one of the plurality of weights;

summing the weighted preliminary baseline estimate and the weighted high resolution mean and subtracting the input physiological waveform to obtain an autoblocked physiological waveform;

weighting the autoblocked physiological waveform with a second one of the plurality of weights;

combining the weighted autoblocked physiological waveform with a final baseline estimate; and

feeding the weighted autoblocked physiological waveform back to the preliminary baseline estimation.

Statement 20. The method of Statement 19, wherein the input physiological waveform is a raw, acquired waveform or a baseline corrected signal.

Statement 21. The method of Statement 19 or 20, wherein the difference between the high resolution mean and the input physiological waveform represents a signal deviation.

Statement 22. The method of any of Statements 19-21, wherein the difference between the high resolution mean and the preliminary baseline estimate represents a mean deviation.

Statement 23. The method of any of Statements 19-22, wherein summing the weighted preliminary baseline estimate and the weighted high resolution mean yields a final baseline estimation.

Statement 24. A physiological waveform monitor, comprising:

a deviation estimating module that, in operation, generates a high resolution mean from an input physiological waveform;

a preliminary baseline estimation module that, in operation, generates a preliminary baseline estimation;

a weight generating module that, in operation, generates a plurality of weights based on signal magnitude from a deviation in the input physiological waveform and a mean deviation in the preliminary baseline estimation;

a signal correction module that, in operation, generates:

an autoblocked physiological waveform from the difference between the input physiological waveform and a final estimated baseline, the final estimated baseline being the combination of the high resolution mean weighted with a first one of the plurality of weights and the preliminary baseline weighted with 1 minus the first one of the plurality of weights;

a baseline corrected signal from the combination of the autoblocked physiological waveform weighted with a second one of the plurality of weights and the final estimated baseline; and

a feedback for the baseline corrected signal to the preliminary baseline estimation module; and

a display that, in operation, displays the autoblocked physiological waveform.

Statement 25. The physiological waveform monitor of Statement 24, wherein the input physiological waveform is a raw, acquired waveform or a baseline corrected signal.

Statement 26. The physiological waveform monitor of Statement 24 or 25, wherein the deviation estimation module further comprises a high resolution, moving average finite impulse response ("FIR") filter with a small window size.

Statement 27. The physiological waveform monitor of Statement 26, wherein the small window size is 0.2 seconds.

Statement 28. The physiological waveform monitor of any of Statements 24-27, wherein generating a high resolution mean from an input physiological waveform includes applying a high resolution, moving average finite impulse response filter with a small window size to the input physiological waveform.

Statement 29. The physiological waveform monitor of Statement 28, wherein the small window size is 0.2 seconds.

Statement 30. The physiological waveform monitor of any of Statements 24-29, wherein the deviation in the input physiological waveform is the difference between the input physiological waveform and a high resolution mean of the input physiological waveform.

Statement 31. The physiological waveform monitor of any of Statements 24-30, wherein the preliminary baseline estimation module further comprises a moving average finite impulse response filter with a large window size.

Statement 32. The physiological waveform monitor of Statement 31, wherein the large window size is 1 to 2 seconds.

Statement 33. The physiological waveform monitor of any of Statements 24-32, wherein generating a preliminary baseline estimation includes applying a moving average finite impulse response filter with a large window size to the correction signal.

Statement 34. The physiological waveform monitor of Statement 33, wherein the large window size is 1 to 2 seconds.

Statement 35. A method for autoblocking a physiological waveform display to mitigate baseline wandering in a physiological waveform substantially as shown and described.

Statement 36. A physiological monitoring device configured to mitigate baseline wandering in a physiological waveform substantially as shown and described.

Statement 37. A local physiological system configured to mitigate baseline wandering in a physiological waveform substantially as shown and described

Statement 38. A larger physiological system configured to mitigate baseline wandering in a physiological waveform substantially as shown and described.

**Claims**

1. A method for autoblocking a physiological waveform display to mitigate baseline wander, comprising:

   extracting a low resolution preliminary baseline estimate from an input physiological waveform;
   extracting a high resolution deviation mean from the input physiological waveform;
   subtracting the low resolution preliminary baseline estimate from the high resolution deviation mean to extract a mean deviation of the input physiological waveform;
   determining a weight factor positively correlated to the mean deviation;
   applying the weight factor to the mean deviation of the input physiological waveform;
   applying the difference between 1 and the weight factor to the preliminary baseline estimate;
   combining the weighted mean deviation of the input physiological waveform with the weighted preliminary baseline estimate to extract an adjusted baseline estimate; and
   subtracting the adjusted baseline estimate from the input physiological waveform to obtain an autoblocked physiological waveform for display.

2. The method of claim 1, wherein the weight factor is between 0 and 1.

3. The method of claim 1 or 2, wherein determining a weight factor positively correlated to the mean deviation includes obtaining the square of the mean deviation divided by sum of mean deviation and a constant.

4. The method of any preceding claim, wherein the constant is 1±10%.

5. The method of any preceding claim, wherein combining the weighted mean deviation of the input physiological waveform with the weighted preliminary baseline estimate suppresses the deviation in the preliminary baseline estimate.

6. The method of any preceding claim, further comprising:

   suppressing the deviation in the autoblocked physiological waveform;
   adding the deviation suppressed autoblocked physiological waveform to the adjusted baseline estimate; and
   feeding the adjusted baseline estimate back to the low resolution preliminary baseline estimate generation.

7. The method of claim 6, wherein suppressing the deviation in the autoblocked physiological waveform further comprises:

   subtracting the input physiological waveform from the high resolution deviation mean to extract a high resolution deviation;
   determining a deviation suppression weight factor by utilizing the mean weight factor and the high resolution deviation with a suppression coefficient; and
   applying the deviation suppression weight factor to the autoblocked physiological waveform.

8. The method of claim 7, wherein the deviation suppression weight factor is between 0 and 1.

9. The method of claim 7 or 8, wherein the suppression coefficient is 10±10%.

10. A physiological waveform monitor, comprising:

    a deviation estimating module that, in operation, generates a high resolution mean from an input physiological waveform;
    a preliminary baseline estimation module that, in operation, generates a preliminary baseline estimation;
    a weight generating module that, in operation, generates a plurality of weights based on signal magnitude from a deviation in the input physiological waveform and a mean deviation in the preliminary baseline estimation;

a signal correction module that, in operation, generates:

    an autoblocked physiological waveform from the difference between the input physiological waveform and a final estimated baseline, the final estimated baseline being the combination of the high resolution mean weighted with a first one of the plurality of weights and the preliminary baseline weighted with 1 minus the first one of the plurality of weights;
    a baseline corrected signal from the combination of the autoblocked physiological waveform weighted with a second one of the plurality of weights and the final estimated baseline; and
    a feedback for the baseline corrected signal to the preliminary baseline estimation module; and

a display that, in operation, displays the autoblocked physiological waveform.

11. The physiological waveform monitor of claim 10, wherein the deviation estimation module further comprises a high resolution, moving average finite impulse response ("FIR") filter with a small window size.

12. The physiological waveform monitor of claim 10 or 11, wherein generating a high resolution mean from an input physiological waveform includes applying a high resolution, moving average finite impulse response filter with a small window size to the input physiological waveform.

13. The physiological waveform monitor of any one of claims 10-12, wherein the deviation in the input physiological waveform is the difference between the input physiological waveform and a high resolution mean of the input physiological waveform.

14. The physiological waveform monitor of any one of claims 10-13, wherein the preliminary baseline estimation module further comprises a moving average finite impulse response filter with a large window size.

15. The physiological waveform monitor of any one of claims 10-14, wherein generating a preliminary baseline estimation includes applying a moving average finite impulse response filter with a large window size to the correction signal.

**FIG. 1**

200

EXTRACT A LOW RESOLUTION
PRELIMINARY BASELINE ESTIMATE FROM — 210
AN INPUT PHYSIOLOGICAL WAVEFORM

⇩

EXTRACT A HIGH RESOLUTION
DEVIATION MEAN FROM THE INPUT — 220
PHYSIOLOGICAL WAVEFORM

⇩

SUBTRACT THE LOW RESOLUTION PRELIMINARY
BASELINE ESTIMATE FROM THE HIGH RESOLUTION — 230
DEVIATION MEAN TO EXTRACT A MEAN DEVIATION OF
THE INPUT PHYSIOLOGICAL WAVEFORM

⇩

DETERMINE A WEIGHT FACTOR POSITIVELY — 240
CORRELATED TO THE MEAN DEVIATION

⇩

APPLY THE WEIGHT FACTOR TO THE MEAN DEVIATION — 250
OF THE INPUT PHYSIOLOGICAL WAVEFORM

⇩

APPLY THE DIFFERENCE BETWEEN 1 AND THE WEIGHT — 260
FACTOR TO THE PRELIMINARY BASELINE ESTIMATE

⇩

COMBINE THE WEIGHTED MEAN DEVIATION OF THE
INPUT PHYSIOLOGICAL WAVEFORM WITH THE — 270
WEIGHTED PRELIMINARY BASELINE ESTIMATE TO
EXTRACT AN ADJUSTED BASELINE ESTIMATE

⇩

SUBTRACT THE ADJUSTED BASELINE
ESTIMATE FROM THE INPUT PHYSIOLOGICAL — 280
WAVEFORM TO OBTAIN AN AUTOBLOCKED
PHYSIOLOGICAL WAVEFORM FOR DISPLAY

# FIG. 2

106

120

104

300

PHYSIOLOGICAL MONITORING DEVICE

PROCESSOR-BASED
RESOURCE

307

302

310

MEMORY

315

INSTRUCTIONS

305

DATA

# FIG. 3

400

SUPPRESS THE DEVIATION IN THE
AUTOBLOCKED PHYSIOLOGICAL WAVEFORM

410

ADD THE DEVIATION SUPPRESSED
AUTOBLOCKED PHYSIOLOGICAL WAVEFORM
TO THE ADJUSTED BASELINE ESTIMATE

420

FEED THE ADJUSTED BASELINE
ESTIMATE BACK TO THE LOW
RESOLUTION PRELIMINARY BASELINE
ESTIMATE GENERATION

430

# FIG. 4

500

```
┌─────────────────────────────────────┐
│  EXTRACT A LOW RESOLUTION            │──── 505
│  PRELIMINARY BASELINE ESTIMATE FROM  │
│  AN INPUT PHYSIOLOGICAL WAVEFORM     │
└─────────────────────────────────────┘
                  ⇩
┌─────────────────────────────────────┐
│  EXTRACT A HIGH RESOLUTION           │──── 510
│  DEVIATION MEAN FROM THE INPUT       │
│  PHYSIOLOGICAL WAVEFORM              │
└─────────────────────────────────────┘
                  ⇩
┌─────────────────────────────────────┐
│  SUBTRACT THE PRELIMINARY BASELINE   │
│  ESTIMATE FROM THE DEVIATION MEAN    │──── 515
│  TO EXTRACT A MEAN DEVIATION OF THE  │
│  INPUT PHYSIOLOGICAL WAVEFORM        │
└─────────────────────────────────────┘
                  ⇩
┌─────────────────────────────────────┐
│  DETERMINE A MEAN WEIGHT FACTOR      │
│  POSITIVELY CORRELATED TO THE MEAN   │──── 520
│  DEVIATION BY OBTAINING THE SQUARE   │
│  OF THE MEAN DEVIATION DIVIDED BY A  │
│  SUM OF THE MEAN DEVIATION AND A     │
│  CONSTANT                            │
└─────────────────────────────────────┘
                  ⇩
┌─────────────────────────────────────┐
│  EXTRACT AN ADJUSTED BASELINE        │
│  ESTIMATE BY SUMMING THE MEAN        │──── 525
│  DEVIATION WEIGHTED WITH THE MEAN    │
│  WEIGHT FACTOR AND THE PRELIMINARY   │
│  BASELINE ESTIMATE WEIGHTED WITH 1   │
│  MINUS THE MEAN WEIGHT FACTOR        │
└─────────────────────────────────────┘
                  ⇩
┌─────────────────────────────────────┐
│  SUBTRACT THE ADJUSTED BASELINE      │
│  ESTIMATE FROM THE INPUT             │──── 530
│  PHYSIOLOGICAL WAVEFORM TO OBTAIN    │
│  THE AN AUTOBLOCKED PHYSIOLOGICAL    │
│  WAVEFORM FOR DISPLAY                │
└─────────────────────────────────────┘
                  ⇩
                 ( A )
```

# FIG. 5A

500

A

SUBTRACT THE INPUT PHYSIOLOGICAL WAVEFORM FROM THE HIGH RESOLUTION DEVIATION MEAN TO EXTRACT A HIGH RESOLUTION DEVIATION — 535

DETERMINE A DEVIATION SUPPRESSION WEIGHT FACTOR BY UTILIZING THE MEAN WEIGHT FACTOR AND THE HIGH RESOLUTION DEVIATION WITH A SUPPRESSION COEFFICIENT — 540

APPLY THE DEVIATION SUPPRESSION WEIGHT FACTOR TO THE AUTOBLOCKED PHYSIOLOGICAL WAVEFORM — 545

SUM THE WEIGHTED AUTOBLOCKED PHYSIOLOGICAL WAVEFORM AND THE ADJUSTED BASELINE ESTIMATE TO OBTAIN A DEVIATION SUPPRESSED CORRECTED SIGNAL — 550

FEED THE DEVIATION SUPPRESSED CORRECTED SIGNAL BACK TO THE LOW RESOLUTION PRELIMINARY BASELINE ESTIMATE GENERATION — 555

FIG. 5B

**FIG. 6**

700

DETERMINE A HIGH RESOLUTION
MEAN OF AN INPUT
PHYSIOLOGICAL WAVEFORM — 710

DETERMINE A PRELIMINARY
BASELINE ESTIMATION — 720

GENERATE A PLURALITY OF WEIGHTS FROM THE
DIFFERENCE BETWEEN THE INPUT PHYSIOLOGICAL
WAVEFORM AND THE HIGH RESOLUTION MEAN AND FROM
THE DIFFERENCE BETWEEN THE HIGH RESOLUTION MEAN
AND THE PRELIMINARY BASELINE ESTIMATE — 730

WEIGHT THE HIGH RESOLUTION MEAN WITH A FIRST ONE
OF THE PLURALITY OF WEIGHTS AND THE PRELIMINARY
BASELINE ESTIMATION WITH THE DIFFERENCE BETWEEN
1 AND THE FIRST ONE OF THE PLURALITY OF WEIGHTS — 740

SUM THE WEIGHTED PRELIMINARY BASELINE ESTIMATE AND
THE WEIGHTED HIGH RESOLUTION MEAN AND SUBTRACTING
THE INPUT PHYSIOLOGICAL WAVEFORM TO OBTAIN AN
AUTOBLOCKED PHYSIOLOGICAL WAVEFORM — 750

WEIGHT THE AUTOBLOCKED PHYSIOLOGICAL
WAVEFORM WITH A SECOND ONE OF THE
PLURALITY OF WEIGHTS — 760

COMBINE THE WEIGHTED AUTOBLOCKED
PHYSIOLOGICAL WAVEFORM WITH A FINAL
BASELINE ESTIMATE — 770

FEED WEIGHTED AUTOBLOCKED
PHYSIOLOGICAL WAVEFORM BACK TO THE
PRELIMINARY BASELINE ESTIMATION — 780

FIG. 7

FIG. 8

FIG. 9

**EUROPEAN SEARCH REPORT**

Application Number

EP 24 19 5176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/018702 A1 (GALLOWAY CONNER DANIEL CROSS [US] ET AL) 15 January 2015 (2015-01-15) * paragraph [0005] - paragraph [0006] * * paragraph [0033] - paragraph [0050] * ----- | 1-15 | INV. A61B5/00 A61B5/318 |
| A | CN 112 603 325 A (UNIV SHANGHAI JIAOTONG) 6 April 2021 (2021-04-06) * paragraph [0053] - paragraph [0054] * ----- | 1-15 | |
| A | RAHUL JAGDEEP ET AL: "Dynamic thresholding based efficient QRS complex detection with low computational overhead", BIOMEDICAL SIGNAL PROCESSING AND CONTROL, vol. 67, 9 March 2021 (2021-03-09), page 102519, XP055885753, NL ISSN: 1746-8094, DOI: 10.1016/j.bspc.2021.102519 * page 3 * ----- | 1-15 | |

TECHNICAL FIELDS SEARCHED (IPC)

A61B

The present search report has been drawn up for all claims

| Place of search | Date of completion of the search | Examiner |
|---|---|---|
| The Hague | 18 November 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 5176

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2024

| Patent document cited in search report | Publication date | Patent family member(s) | Publication date |
|---|---|---|---|
| US 2015018702 A1 | 15-01-2015 | US 2015018702 A1<br>US 2016242665 A1<br>US 2017215756 A1 | 15-01-2015<br>25-08-2016<br>03-08-2017 |
| CN 112603325 A | 06-04-2021 | CN 112603325 A<br>CN 113180685 A | 06-04-2021<br>30-07-2021 |

For more details about this annex : see Official Journal of the European Patent Office, No. 12/82